# EUROPÄISCHE PATENTSCHRIFT

(11) **EP 0 812 781 B1**
(45) Veröffentlichungstag und Bekanntmachung des Hinweises auf die Patenterteilung: **16.06.1999**
(21) Anmeldenummer: 96116839.0
(22) Anmeldetag: 19.10.1996
(51) Int. Cl.: B65D 75/42, A61F 5/11

(54) **Verpackungsanordnung für Nagelkorrekturstreifen**
Packaging system for nail correcting strips
Système d'emballage pour bandes de corrections d'ongles

(30) Priorität: 11.06.1996 DE 29610208 U
(43) Veröffentlichungstag der Anmeldung: 17.12.1997
(73) Patentinhaber: Bernd Stolz GmbH, 92224 Amberg (DE)
(72) Erfinder: Stolz, Bernd, 92224 Amberg (DE)
(74) Vertreter: Schneck, Herbert, Dipl.-Phys., Dr.

(56) Entgegenhaltungen:
- EP-A- 0 282 645
- FR-A- 2 502 114
- US-A- 3 799 160
- US-A- 4 735 342

## Beschreibung

Die Erfindung richtet sich auf eine Verpackungsanordnung für Nagelkorrekturstreifen.

Nagelkorrekturstreifen der in Betracht stehenden Art sind aus dem europäischen Patent 0 282 645 bekannt, das als Grundlage für den Oberbegriff das Anspruchs 1 dient. Derartige Nagelkorrekturstreifen dienen dazu, stark gekrümmte, seitlich eingewachsene Fußnägel wieder aufzurichten und die durch die Krümmung verursachten Schmerzen zu beseitigen.

Zu diesem Zweck sind Kunststoff-Streifen mit einer vergleichsweise hohen elastischen Rückstellkraft entwickelt worden, welche auf der Oberfläche des zu korrigierenden Nagels mittels eines Schnellklebers befestigt werden und dementsprechend die eigenelastischen Rückstellkräfte dauerhart auf den Nagel übertragen, so daß die Ränder wieder in Richtung auf eine Korrektur der Krümmung begradigt werden.

Die Handhabung von Schnellklebern erfordert ein hohes Maß an Aufmerksamkeit, da aufgrund von deren hoher Klebkraft die Gefahr besteht, daß die Finger der handhabenden Person verlieben oder der Klebstoff anderweitig Schäden anrichtet.

Hiervon ausgehend liegt der Erfindung die Aufgabe zugrunde, eine Verpackungsanordnung der eingangs genannten Art so auszugestalten, daß sie einerseits eine raumsparende Lagerung und einen kostengünstigen Versand, andererseits aber auch eine vereinfachte Handhabung beim Anbringen an dem Nagel ermöglicht.

Diese Aufgabe wird durch eine Verpackungsanordnung gemäß Anspruch 1 gelöst.

Durch diese Ausgestaltung ist es möglich, den Klebstoff auf den Korrekturstreifen aufzubringen, ohne daß der Korrekturstreifen selbst in der Hand gehalten werden müßte. Vielmehr eröffnet der Träger eine sehr einfache und bequeme Handhabungsmöglichkeit. Ist der Korrekturstreifen mit dem Klebstoff versehen, wird er mit der klebstoffbeschichteten Seite auf den Nagel aufgelegt. Die beiderseits überstehenden Handhabungsabschnitte des Trägers ermöglichen es, über den Träger Druck auf den Korrekturstreifen auszuüben und, wenn dieser positioniert ist, den Träger abzuziehen. Der Träger ist mit dem Korrekturstreifen über Adhäsion oder über einen drucksensitiven Kleber niedriger Klebekraft verbunden, so daß diese einerseits eine sichere Handhabung der Kombination aus Träger und Korrekturstreifen ermöglicht, andererseits aber gewährleistet, daß der auf den Nagel aufgebrachte Korrekturstreifen dort beim Abziehen des Trägers auch sicher verbleibt.

In weiterer Ausgestaltung der Erfindung kann vorgesehen sein, daß Träger und Korrekturstreifen mit einer Abdeckfolie überzogen sind, wobei diese Schutzfolie dann vor dem Aufbringen entfernt wird.

Günstigerweise kann weiterhin vorgesehen sein, daß mehrere Träger als Folienbogen oder Pappebogen zusammengefaßt sind und vor der Benutzung jeweils ein Trägerstreifen mit je einem Korrekturstreifen hiervon über eine Perforation oder durch Abschneiden abgetrennt wird.

In weiterer Ausgestaltung der Erfindung ist vorgesehen, daß auf dem Träger ein Abstandshalte-Streifen angeordnet ist, auf welchem wiederum der Nagelkorrektur-Streifen ruht. Hierdurch wird erreicht, daß der Nagelkorrektur-Streifen vom Träger abgehoben ist, wodurch sich das Aufbringen, ohne daß die Finger mit Klebstoff in Berührung kommen, noch wesentlich vereinfacht.

Vorzugsweise besteht der Abstandshalte-Streifen aus einem elastischen Material, insbesondere aus einem elastischen Kunststoff.

Der Nagelkorrektur-Streifen ist mit dem Abstandshalte-Streifen klebend derart verbunden, daß beim Aufdrücken des Nagelkorrektur-Streifens auf den zu korrigierenden, mit Klebstoff beschichteten Nagel der Nagelkorrektur-Streifen am Nagel verbleibt, weil die Klebekraft zwischen ihm und dem Abstandshalte-Streifen entsprechend gering eingestellt ist. Diese Klebekraft kann durch einen Klebstoff oder beispielsweise auch durch Adhäsion realisiert sein.

Gemäß einem weiteren vorteilhaften Merkmal der Erfindung entspricht die Breite des Trägers der Länge des Nagelkorrektur-Streifens, der darauf angeordnet ist. Dementsprechend kann die handhabende Person mit Hilfe der Breite des Trägers am Nagel des Patienten ausmessen, ob die Länge des vorgesehenen Nagelkorrektur-Streifens richtig ist und gegebenenfalls eine andere Korrekturstreifen-Größe wählen, ohne hierzu den Nagelkorrektur-Streifen vom Träger entfernen zu müssen.

Nachfolgend wird die Erfindung anhand eines bevorzugten Ausführungsbeispiels in Verbindung mit der Zeichnung näher beschrieben. Dabei zeigen:
- Fig. 1: eine Aufsicht auf eine mehrere Trägerstreifen umfassende Trägereinheit,
- Fig. 2: einen Schnitt längs der Linie II-II in Fig. 1 und
- Fig. 3: einen Fig. 2 entsprechenden Schnitt einer zweiten Ausführungsform.

In der Zeichnung ist eine als Pappbogen ausgebildete Trägereinheit 1 dargestellt, welche mehrere Träger 2 umfaßt, die zunächst miteinander verbunden sind und, wie durch das Scherensymbol 3 angedeutet, im Bedarfsfall voneinander separiert werden können.

Auf jedem Träger 2 ist ein Korrekturstreifen 4 aus Kunststoff angeordnet, wobei jeder Träger 2 und jeder Korrekturstreifen 4 von einer Abdeckfolie 5 überzogen ist.

Im Gebrauch wird aus der Trägereinheit 1 ein streifenförmiger Träger 2 abgeschnitten und die Schutzfolie 5 entfernt.

Es wird dann die Oberseite 6 des Korrekturstreifens 4 mit einem Schnellkleber bestrichen und der Träger an den seitlichen Halteabschnitten 7 mit zwei Händen erfaßt, gewendet und die beschichtete Oberseite 6 des Korrekturstreifens 4 auf den zu korrigierenden Nagel aufgedrückt. Nach erfolgter Positionierung wird der Träger 2 abgezogen und der Korrekturstreifen 4 sitzt auf dem Nagel, ohne daß die handhabende Person Gefahr läuft, mit dem Klebstoff in Berührung zu kommen.

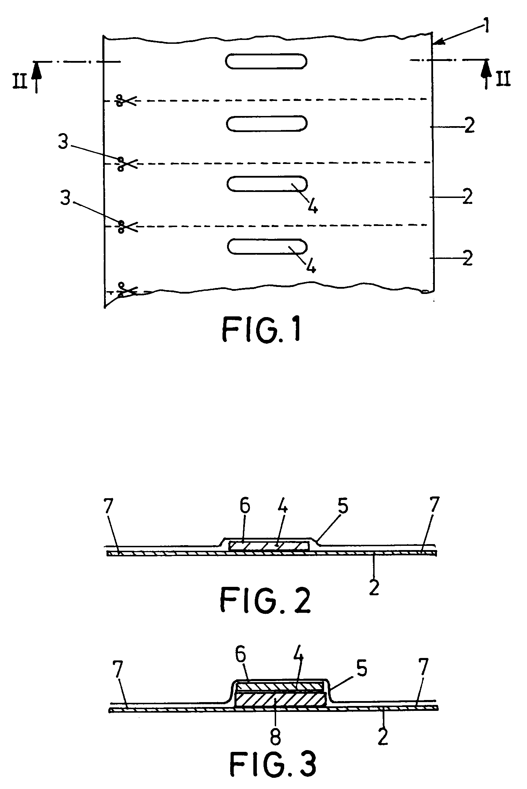

Bei der in Fig. 3 dargestellten Ausführungsform ist zusätzlich zwischen dem Träger 2 und dem Nagelkorrektur-Streifen 4 ein Abstandshalte-Streifen 8 aus elastischem Kunststoff angeordnet, der dafür sorgt, daß der Nagelkorrektur-Streifen 4 bei der Applikation vorsteht und dementsprechend noch problemloser angeklebt werden kann, ohne daß die Gefahr besteht, daß die Finger der handhabenden Person mit auf dem Nagel angebrachten Klebstoff in Berührung kommt.

## Patentansprüche

1. Verpackungsanordnung mit Nagelkorrekturstreifen, wobei der Nagelkorrekturstreifen (4) eine vergleichsweise hohe elastische Rückstellkraft aufweist und mittels eines Schnellklebers auf der Oberseite eines eingewachsenen Zehnagels befestigbar ist, dadurch gekennzeichnet, daß jeder Nagelkorrekturstreifen (4) auf einem folien- bzw. kartonartigen Träger (2) angeordnet ist, der den eigentlichen Korrekturstreifen (4) seitlich überragt, und, daß der Nagelkorrekturstreifen (4) mit dem Träger (2) über Adhäsion oder über einen drucksensitiven Kleber niedriger Klebekraft verbunden ist.

2. Verpackungsanordnung nach Anspruch 1, dadurch gekennzeichnet, daß Träger (2) und Korrekturstreifen (4) von einer Schutzfolie (5) überzogen sind.

3. Verpackungsanordnung nach Anspruch 1, dadurch gekennzeichnet, daß mehrere Träger (2) als Folien- oder Pappebogen zusammengefaßt sind und vor der Benutzung jeweils ein Träger-Streifen mit je einem Korrekturstreifen (4) hiervon über eine Perforation oder durch Abschneiden abtrennbar ist.

4. Verpackungsanordnung nach Anspruch 1, dadurch gekennzeichnet, daß auf dem Träger (2) ein Abstandshalte-Streifen (8) angeordnet ist, auf welchem wiederum der Nagelkorrektur-Streifen (4) ruht.

5. Verpackungsanordnung nach Anspruch 4, dadurch gekennzeichnet, daß der Abstandshalte-Streifen (8) aus einem elastischen Material, insbesondere aus einem elastischen Kunststoff, besteht.

6. Verpackungsanordnung nach Anspruch 4, dadurch gekennzeichnet, daß der Nagelkorrektur-Streifen (4) mit dem Abstandshalte-Streifen (8) klebend derart verbunden ist, daß beim Aufdrücken des Nagelkorrektur-Streifens (4) auf den zu korrigierenden, mit Klebstoff beschichteten Nagel der Nagelkorrektur-Streifen (4) am Nagel verbleibt aufgrund der niedrig eingestellten Klebekraft zwischen ihm und dem Abstandshalte-Streifen (8).

7. Verpackungsanordnung nach Anspruch 1, dadurch gekennzeichnet, daß die Breite des Trägers (2) der Länge des darauf angeordneten Nagelkorrektur-Streifens (4) entspricht.

## Claims

1. A packaging arrangement comprising nail correction strips, the nail correction strip (4) having a comparatively high elastic restoring force and being fastenable on the surface of an in-grown toe nail by means of an instantaneous adhesive, characterized in that each nail correction strip (4) is arranged on a film- or cardboard-like carrier (2) projecting over the sides of the actual correction strip (4) and in that the nail correction strip (4) is connected with the carrier (2) by means of adhesion or by means of a pressure-sensitive adhesive of low adhesive strength.

2. A packaging arrangement according to claim 1, characterized in that the carrier (2) and the correction strip (4) are covered by a protective film (5).

3. A packaging arrangement according to claim 1, characterized in that several carriers (2) are combined as a sheet of film or as a sheet of cardboard and that before each use, a carrier strip together with a correction strip (4) is detachable by way of a perforation or by being cut off.

4. A packaging arrangement according to claim 1, characterized in that the carrier (2) is provided with a spacer strip (8), on which in turn rests the nail correction strip (4).

5. A packaging arrangement according to claim 4, characterized in that the spacer strip (8) consists of an elastic material, in particular of an elastic plastic material.

6. A packaging arrangement according to claim 4, characterized in that the nail correction strip (4) is adhesively connected with the spacer strip (8) such that when pressed on the nail to be corrected and coated with adhesive, the nail correction strip (4) remains on the nail due to the low adjustment of the adhesive strength between the nail correction strip and the spacer strip (8).

7. A packaging arrangement according to claim 1, characterized in that the width of the carrier (2) corresponds to the length of the nail correction strip (4) arranged thereon.

## Revendications

1. Système d'emballage comportant des bandes de corrections d'ongles, la bande (4) de correction d'ongle présentant une force de rappel élastique relativement grande et pouvant être fixée, au moyen d'un adhésif à adhérence rapide, sur la face supérieure d'un ongle d'orteil incarné, caractérisé par le fait que chaque bande (4) de correction d'ongle est disposée sur un support (2) du type film ou carton, faisant saillie latéralement au-delà de ladite bande de correction (4) proprement dite ; et par le fait que la bande (4) de correction d'ongle est reliée au support (2) par adhésion, ou par l'intermédiaire d'un adhésif autocollant à force d'adhérence modeste.

2. Système d'emballage selon la revendication 1, caractérisé par le fait que le support (2) et la bande de correction (4) sont recouverts d'un film protecteur (5).

3. Système d'emballage selon la revendication 1, caractérisé par le fait que plusieurs supports (2) sont regroupés sous la forme de feuilles en film ou en carton dont une bande de support, respectivement munie d'une bande de correction (4), peut être à chaque fois séparée avant l'utilisation, par l'intermédiaire d'une perforation ou par sectionnement.

4. Système d'emballage selon la revendication 1, caractérisé par le fait qu'une bande d'espacement (8), sur laquelle la bande (4) de correction d'ongle repose à son tour, est disposée sur le support (2).

5. Système d'emballage selon la revendication 4, caractérisé par le fait que la bande d'espacement (8) consiste en un matériau élastique, notamment en une matière plastique élastique.

6. Système d'emballage selon la revendication 4, caractérisé par le fait que la bande (4) de correction d'ongle est reliée par adhérence à la bande d'espacement (8) d'une manière telle que, lorsque ladite bande (4) de correction d'ongle est pressée contre l'ongle à corriger, revêtu d'adhésif, ladite bande (4) de correction d'ongle demeure sur ledit ongle du fait que la force d'adhérence est réglée à une faible valeur entre cette bande et la bande d'espacement (8).

7. Système d'emballage selon la revendication 1, caractérisé par le fait que la largeur du support (2) correspond à la longueur de la bande (4) de correction d'ongle, placée sur ledit support.
